# FASCICULE DE BREVET EUROPEEN

(11) **EP 2 717 780 B1**
(45) Date de publication et mention de la délivrance du brevet: **25.04.2018**
(21) Numéro de dépôt: 12729078.1
(22) Date de dépôt: 07.06.2012
(51) Int. Cl.: A61B 8/12, A61B 5/00, A61B 8/00

(54) **SONDE DE DIAGNOSTIC BIMODALE A IMAGERIES OPTIQUE ET ULTRASONORE, COMPRENANT AU MOINS UNE COQUE DEMONTABLE A MOYENS OPTIQUES EMBARQUES**
BIMODALE DIAGNOSTISCHE SONDE MIT VERWENDUNG VON OPTISCHER UND ULTRASCHALL-BILDGEBUNG MIT MINDESTENS EINER ABNEHMBAREN VERKLEIDUNG MIT BORDEIGENER OPTISCHER VORRICHTUNG
BIMODAL DIAGNOSTIC PROBE USING OPTICAL AND ULTRASONIC IMAGING, INCLUDING AT LEAST ONE REMOVABLE SHELL HAVING ON-BOARD OPTICAL MEANS

(30) Priorité: 07.06.2011 FR 1154960
(43) Date de publication de la demande: 16.04.2014
(73) Titulaire: Commissariat à l'Énergie Atomique et aux Énergies Alternatives, 75015 Paris (FR); SA Vermon, 37000 Tours (FR)
(72) Inventeur: BOUTET, Jérôme, 38640 Claix (FR); NGUYEN-DINH, An, 37520 La Riche (FR); DEBOURDEAU, Mathieu, 38830 Saint Pierre d'Allevard (FR); MESSINEO, Odile, 41000 Blois (FR); NOTARD, Christophe, 37150 La Croix En Touraine (FR)
(74) Mandataire: Ahner, Philippe
(86) Numéro de dépôt international: PCT/EP2012/060815
(87) Numéro de publication internationale: WO 2012/168376

(56) Documents cités:
- WO-A1-2007/147262
- BOUTET J ET AL: "De la lumire infrarouge pour guider les biopsies de la prostate", IRBM, ELSEVIER, AMSTERDAM, NL, vol. 32, no. 2, 23 janvier 2011 (2011-01-23), pages 123-125, XP028159431, ISSN: 1959-0318, DOI: 10.1016/J.IRBM.2011.01.020 [extrait le 2011-02-01]

## Description

### DOMAINE TECHNIQUE

La présente invention se rapporte au domaine des sondes de diagnostic bimodales à imageries optique et ultrasonore.

Elle concerne tout particulièrement les sondes bimodales endorectales, destinées au diagnostic du cancer de la prostate. L'invention peut également concerner les sondes vaginales, ou bien être employée aux fins de tout autre diagnostic susceptible de requérir une imagerie optique et une imagerie ultrasonore.

### ÉTAT DE LA TECHNIQUE ANTÉRIEURE

Les sondes bimodales à imageries optique et ultrasonore ont été récemment développées, notamment dans le domaine de la détection du cancer de la prostate. Un exemple de réalisation est décrit dans le document « Bimodal Ultrasound and Fluorescence Approch for Prostate Cancer Diagnosis », Journal of Biomedical Optics 14(6), 2009).

Ce type de sonde se révèle performant en raison de la complémentarité des deux modalités, l'imagerie optique étant capable d'apporter une information fonctionnelle avec un bon contraste, tandis que l'imagerie ultrasonore apporte une information morphologique avec une bonne résolution.

Ainsi, dans le diagnostic précoce de certaines pathologies, l'approche consiste, à l'aide de mesures optiques et ultrasonores, à réaliser une première localisation des tumeurs potentielles, afin de guider ensuite un outil de biopsie destiné à prélever un échantillon de tissu dans la ou les zones suspectes. Une analyse de ces tissus sous microscope permet par la suite de confirmer ou pas leur nature tumorale.

Le document "De la lumière infrarouge pour guider les biopsies de la prostate", par Boutet et al., IRBM 32(2011), 123-125, décrit une sonde de diagnostic bimodale à imagerie optique et ultrasonore pour guider les biopsies de la prostate.

Si de telles sondes donnent entière satisfaction dans les fonctionnalités qu'elles procurent, leur conception reste néanmoins à optimiser. En particulier, l'accès aux équipements optiques peut être compliqué, et nécessiter l'intervention d'un technicien spécialisé pour opérer des réparations et/ou des changements d'équipements. En outre, cela complexifie la fabrication de telles sondes.

### EXPOSÉ DE L'INVENTION

L'invention a donc pour but de remédier au moins partiellement aux inconvénients mentionnés ci-dessus, relatifs aux réalisations de l'art antérieur.

Pour ce faire, l'invention a tout d'abord pour objet une sonde de diagnostic bimodale à imageries optique et ultrasonore, comportant un corps principal portant à son extrémité avant au moins un transducteur à ultrasons, ladite sonde comprenant en outre au moins un dispositif comportant une coque s'étendant selon une direction longitudinale entre une extrémité arrière et une extrémité avant destinée à former au moins en partie l'extrémité avant de ladite sonde, ledit dispositif comprenant également des moyens d'éclairement montés sur ladite extrémité avant de la coque de manière à assurer un éclairement à l'extérieur de celle-ci, ainsi que des moyens de collecte ou de détection montés sur ladite extrémité avant de la coque de manière à assurer la collecte ou la détection d'un signal optique produit extérieurement à ladite coque,

Selon l'invention, la coque comporte des moyens de fixation permettant son assemblage réversible autour du corps principal de la sonde, avec les moyens d'éclairement et moyens de collecte ou de détection montés sur cette coque.

L'invention est donc remarquable en ce qu'elle prévoit une coque amovible avec des moyens optiques embarqués, ce qui simplifie la conception de la sonde bimodale sur laquelle le dispositif est destiné à être assemblé.

En effet, les réparations et/ou les changements d'équipements optiques embarqués sur la coque peuvent être initiés simplement en démontant cette même coque de la sonde, ce qui procure un accès direct aux équipements optiques, sans avoir à intervenir sur les moyens acoustiques restant quant à eux à poste sur la sonde. Ces opérations peuvent par conséquent être réalisées de manière aisée et rapide, sans nécessiter l'intervention d'un technicien spécialisé.

La conception simplifiée de la présente invention permet également de faciliter la fabrication de la sonde bimodale, dont les moyens acoustiques et les moyens optiques peuvent être parfaitement dissociés. A titre d'exemple, les moyens optiques peuvent être facilement implantés sur leur coque associée, par exemple par insertion et/ou collage.

Par ailleurs, grâce à cette conception, les phases de nettoyage, de désinfection et de stérilisation de la sonde peuvent être mises en oeuvre facilement, en démontant la/les coques à moyens optiques embarqués.

Enfin, il est indiqué que les moyens de fixation réversibles peuvent être de toute conception connue de l'homme du métier. A titre d'exemple, il peut s'agir d'un assemblage par emboîtement, coincement, clipsage, vissage, etc.

De préférence, lesdits moyens d'éclairement sont associés à un premier câblage, lesdits moyens de collecte ou de détection sont associés à un second câblage, et lesdits premier et second câblages cheminent longitudinalement le long de la ladite coque, depuis ladite extrémité avant de la coque, au moins jusqu'à son extrémité arrière. De préférence, ces câblages sont également montés sur la coque, tout le long de celle-ci. Par conséquent, ils accompagnent eux aussi la coque lors de son montage sur la sonde autour du corps principal, et également lors du démontage.

Ces câblages peuvent être de nature optique ou électrique, en fonction de la conception retenue pour les moyens d'éclairement et les moyens de collecte ou de détection montés sur l'extrémité avant de la coque.

A cet égard, il est noté que les moyens d'éclairement sont destinés à produire de la lumière. Il peut par exemple s'agir d'une diode laser, ou encore d'une LED ou d'un élément similaire. Lorsque l'un des éléments de ce type est retenu pour former tout ou partie des moyens d'éclairement, il est monté directement à l'extrémité avant de la coque. Son câblage associé correspond alors à un câblage électrique, permettant de relier cet élément à une source d'énergie électrique.

Alternativement, tout ou partie des moyens d'éclairement peuvent être réalisés à l'aide de l'extrémité d'une fibre optique, dite fibre optique d'excitation, qui constitue alors ledit câblage associé. Dans un tel cas, cette fibre optique d'excitation chemine le long de la coque pour ensuite rejoindre une source de lumière distante, qui peut être pulsée ou continue.

Pour ce qui concerne les moyens de détection, ils sont destinés à détecter de la lumière, c'est-à-dire à détecter un signal optique. Il peut par exemple s'agir d'une photodiode, d'un capteur optique matriciel, ou de tout autre élément similaire. Lorsque l'un des éléments de ce type est retenu pour former tout ou partie des moyens de détection, il est monté directement à l'extrémité avant de la coque. Son câblage associé correspond alors à un câblage électrique, permettant de relier cet élément à une source d'énergie électrique.

Alternativement, il peut être retenu des moyens de collecte, capable de capter de la lumière en vue de l'acheminer vers des moyens de détection distants, par exemple du type de ceux mentionnés ci-dessus. Il peut alors s'agir de l'extrémité d'une fibre optique, la fibre constituant alors ledit câblage associé, en permettant l'acheminement du signal optique vers les moyens de détection déportés. La fibre optique peut néanmoins être remplacée par tout autre guide de lumière réputé approprié pour l'homme du métier, sans sortir du cadre de l'invention.

Préférentiellement, comme évoqué ci-dessus, ledit premier câblage comprend au moins une première fibre optique dont l'extrémité avant, montée sur l'extrémité avant de la coque, forme lesdits moyens d'éclairement, et ledit second câblage comprend au moins une seconde fibre optique dont l'extrémité avant, montée sur l'extrémité avant de la coque, forme lesdits moyens de collecte.

Encore plus préférentiellement, ledit premier câblage comprend une pluralité de premières fibres optiques dont chacune des extrémités avant forme des moyens d'éclairement, ledit second câblage comprend une pluralité de secondes fibres optiques dont chacune des extrémités avant forme des moyens de collecte, les extrémités avant des premières et secondes fibres optiques s'inscrivant sur au moins une ligne courbe, et de préférence une seule, s'inscrivant dans un plan.

De plus, pour une performance optimale, les extrémités avant des premières et secondes fibres optiques sont de préférence agencées en alternance sur une ligne courbe, qui est préférentiellement un arc de cercle.

Par ailleurs, l'écart angulaire entre l'extrémité avant d'une première fibre quelconque et chacune des extrémités avant des deux secondes fibres directement consécutives, est compris entre 5° et 25°, de préférence entre 10 et 20°, et est encore plus préférentiellement de l'ordre de 15°. Ces valeurs, contrastant largement par rapport à celles habituellement retenues dans l'art antérieur, permettent d'améliorer considérablement la collecte du signal optique. Les performances de la sonde sont par conséquent sensiblement accrues.

De préférence, lesdits moyens d'éclairement et lesdits moyens de collecte ou de détection affleurent la surface extérieure de la coque. L'absence de protubérance permet d'éviter des lésions sur les tissus contre lesquels la sonde est destinée à être appliquée. Alternativement, ces moyens pourraient être disposés en retrait de la surface extérieure de la coque, en prévoyant alors de combler l'orifice subsistant d'un matériau transparent, translucide ou diffusant, de telle sorte que l'extrémité avant de la coque présente le moins d'aspérités possible.

De préférence, la sonde comprend au moins deux dispositifs, avec les coques formant conjointement une enveloppe tout autour du corps principal de sonde. De plus, à leurs extrémités avant, lesdites coques définissent un espace comblé par ledit au moins un transducteur à ultrasons. Comme cela sera indiqué ci-après, le nombre de dispositifs est préférentiellement de deux, mais peut être supérieur. Dans tous les cas, les coques des dispositifs sont agencées de manière adjacente selon la direction circonférentielle de la sonde.

La sonde comporte donc préférentiellement deux dispositifs, avec les deux coques étant agencées de manière symétrique selon un plan passant par un axe longitudinal de la sonde.

Cette conception à deux ou plusieurs coques permet de faciliter l'implantation des moyens optiques sur ces mêmes coques, par exemple un prévoyant des moyens de réception des moyens optiques directement sur la surface intérieure de ces coques, par exemple une ou plusieurs rainures ouvertes sur cette surface intérieure. Bien entendu, cette solution à plusieurs coques, dont celle à deux « demi-coques » complémentaires, facilite également les opérations de réparation et/ou de changement des équipements optiques embarqués sur la coque.

De préférence, dans le cas préférentiel de deux dispositifs, chaque demi-coque est globalement concave, la concavité étant orientée vers l'axe longitudinal de la sonde.

Avec les conceptions précédemment décrites, les coques définissent quasiment toute la partie de la sonde destinée à être introduite dans le corps humain lors de la réalisation du diagnostic, à l'exception de la partie recouvrant les moyens acoustique. D'ailleurs, alternativement, ces coques pourraient également recouvrir ledit au moins un transducteur à ultrasons, à condition d'être transparentes aux ultrasons, sans sortir du cadre de l'invention.

Selon un autre mode de réalisation envisagé, la sonde bimodale n'est équipée que d'une seule coque monobloc, qui s'étend donc sur 360°. Les opérations de nettoyage et de stérilisation s'en trouvent simplifiées. De plus, avec la coque monobloc, la sonde s'avère plus rigide.

De préférence, pour la solution à plusieurs coques, la sonde bimodale comprend également, aux interfaces entre les coques, un matériau de remplissage de ces interfaces. Cela permet de faciliter la stérilisation de la sonde, sans nécessairement désassembler ses coques à moyens optiques embarqués. Naturellement, ce matériau de remplissage biocompatible, du type silicone / élastomère, est retenu de façon à ne pas trop contraindre un désassemblage ultérieur des coques de la sonde.

De préférence, la sonde bimodale comprend également un manche de préhension à partir duquel le corps principal s'étend vers l'avant, ledit manche présentant une ou plusieurs rainures sur sa surface extérieure, recevant les premier et second câblages issus de l'extrémité arrière de la coque de chaque dispositif.

Enfin, il est noté que la sonde est préférentiellement une sonde endorectale, destinée au diagnostic du cancer de la prostate. Alternativement, il peut s'agir d'une sonde vaginale.

L'invention a aussi pour objet un procédé de fabrication d'une sonde bimodale telle que décrite ci-dessus, comprenant les étapes suivantes :
- équiper la coque de chaque dispositif de ses moyens d'éclairement et moyens de collecte ou de détection ; puis
- assembler, de manière réversible, chaque dispositif autour du corps principal de la sonde.

Le terme fabrication est ici à considérer au sens large, car il concerne non seulement la fabrication d'une telle sonde bimodale, mais également sa réparation nécessitant la mise en oeuvre des deux étapes mentionnées ci-dessous, et également la transformation d'une sonde ultrasonore en une sonde bimodale, toujours par mise en oeuvre des deux étapes mentionnées ci-dessous. Dans ce dernier cas de la transformation d'une sonde à imagerie ultrasonore en une sonde bimodale selon l'invention, une première étape peut consister à retirer la coque d'origine de la sonde à imagerie ultrasonore.

Enfin, l'invention a pour objet un dispositif pour sonde de diagnostic bimodale à imageries optique et ultrasonore, cette sonde étant destinée à comporter un corps principal portant à son extrémité avant au moins un transducteur à ultrasons. Le dispositif comprend une coque s'étendant selon une direction longitudinale entre une extrémité arrière et une extrémité avant destinée à former au moins en partie l'extrémité avant de la sonde, ce dispositif comprenant également des moyens d'éclairement montés sur l'extrémité avant de la coque de manière à assurer un éclairement à l'extérieur de celle-ci, ainsi que des moyens de collecte ou de détection montés sur l'extrémité avant de la coque de manière à assurer la collecte ou la détection d'un signal optique produit extérieurement à la coque.

Selon l'invention, la coque comporte des moyens de fixation permettant son assemblage réversible autour du corps principal de la sonde, avec les moyens d'éclairement et moyens de collecte ou de détection montés sur cette coque.

De plus, lesdits moyens d'éclairement sont associés à un premier câblage, lesdits moyens de collecte ou de détection sont associés à un second câblage, et lesdits premier et second câblages cheminent longitudinalement le long de la ladite coque, depuis ladite extrémité avant de la coque, au moins jusqu'à son extrémité arrière.

En outre, ledit premier câblage comprend une pluralité de premières fibres optiques dont chacune des extrémités avant forme des moyens d'éclairement, ledit second câblage comprend une pluralité de secondes fibres optiques dont chacune des extrémités avant forme des moyens de collecte, et les extrémités avant des premières et secondes fibres optiques s'inscrivent sur au moins une ligne courbe, qui est un arc de cercle.

De plus, l'écart angulaire entre l'extrémité avant d'une première fibre quelconque et chacune des extrémités avant des deux secondes fibres directement consécutives, est compris entre 5° et 25°, de préférence entre 10 et 20°, et est encore plus préférentiellement de l'ordre de 15°. Ces valeurs, contrastant largement par rapport à celles habituellement retenues dans l'art antérieur, permettent d'améliorer considérablement la collecte du signal optique. Les performances de la sonde associée sont par conséquent sensiblement accrues.

D'autres avantages et caractéristiques de l'invention apparaîtront dans la description détaillée non limitative ci-dessous.

### BRÈVE DESCRIPTION DES DESSINS

Cette description sera faite au regard des dessins annexés parmi lesquels ;
- la figure 1 représente une vue en perspective d'une sonde de diagnostic bimodale selon un mode de réalisation préféré de la présente invention ;
- la figure 2 est une vue éclatée de celle montrée sur la figure 1 ;
- la figure 3 représente une vue de côté de la sonde montrée sur les figures précédentes, accouplée à un système d'imagerie ;
- la figure 4 représente en coupe longitudinale de l'un des deux dispositifs amovibles à coque équipée de moyens optiques, prévus sur la sonde représentée sur les figures précédentes ;
- la figure 5 représente une vue éclatée en perspective d'une partie avant de la sonde montrée sur les figures précédentes ;
- la figure 6 représente une vue en coupe prise le long de la ligne VI-VI de la figure 4 ;
- la figure 7 représente une vue de dessus de la sonde montrée sur les figures précédentes ;
- la figure 8 représente une vue schématique de côté montrant l'agencement des moyens optiques sur l'extrémité avant de la coque montrée sur les figures 4 et 5 ;
- la figure 9 représente une vue schématisant le montage des deux dispositifs amovibles à coque équipée de moyens optiques, sur la sonde ; et
- la figure 10 représente une vue schématique en perspective de la sonde montrée sur les figures précédentes, sur laquelle est monté un outil de biopsie destiné à prélever un échantillon de tissu.

### EXPOSÉ DÉTAILLÉ DE MODES DE RÉALISATION PARTICULIERS

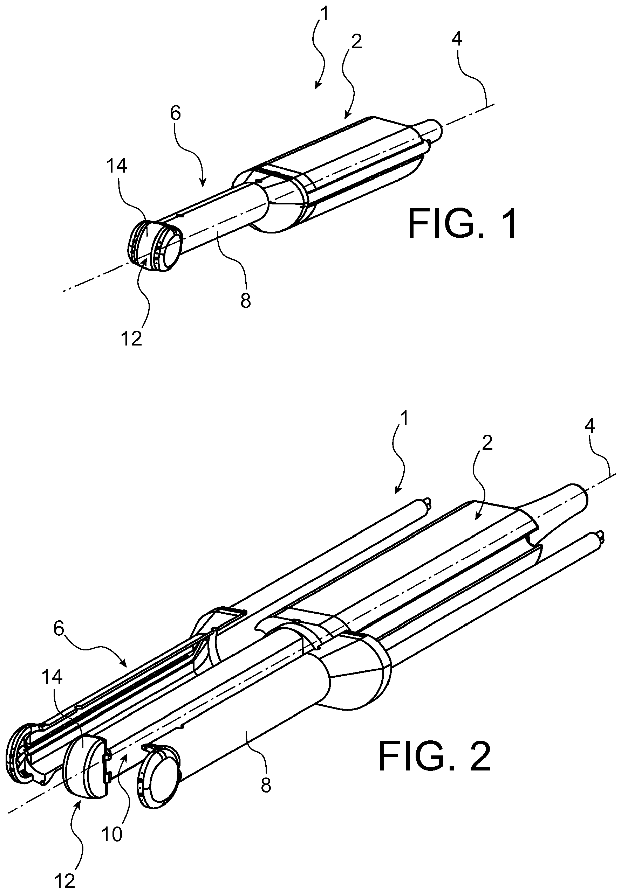

Tout d'abord en référence aux figures 1 à 3, on peut apercevoir une sonde endorectale 1 selon un mode de réalisation préféré de la présente invention.

Cette sonde de diagnostic est bimodale, en ce sens qu'elle est à imagerie optique et à imagerie ultrasonore. Elle comporte un manche de préhension 2 de forme longiligne, d'axe 4 définissant la direction longitudinale de la sonde. Le manche 2 est prolongé vers l'avant, dans la direction de l'axe 4, par une partie d'introduction 6 destinée à être insérée dans la région anatomique à investiguer.

La partie d'introduction 6 présente ainsi une surface extérieure lisse 8, essentiellement formée par deux coques amovibles à moyens optiques embarqués, qui seront explicitées ci-après. Intérieurement, cette partie d'introduction 6 est formée par un corps principal 10 longiligne, de forme sensiblement cylindrique d'axe 4. A son extrémité arrière, le corps principal 10 est porté par le manche 2, tandis que son extrémité avant porte des moyens acoustiques 12, prenant la forme d'un ou plusieurs transducteurs à ultrasons recouverts à l'avant par une protection 14 en matériau du type silicone, formant partie de ladite surface extérieure lisse 8. Cette protection 14 est naturellement transparente aux ultrasons, biocompatible, et assure l'étanchéité de la sonde.

Le ou les transducteurs ultrasonores peuvent être de forme linéaire ou matricielle, avec une géométrie de surface plane, convexe ou concave. Dans le mode de réalisation préféré représenté, le transducteur est un réseau linéaire de géométrie de surface convexe, fournissant un angle de vision sectorielle. En effet, le transducteur 14 prend ici une forme globale de demi-disque dont la face plane est agencée orthogonalement à l'axe 4, en direction de l'arrière de la sonde.

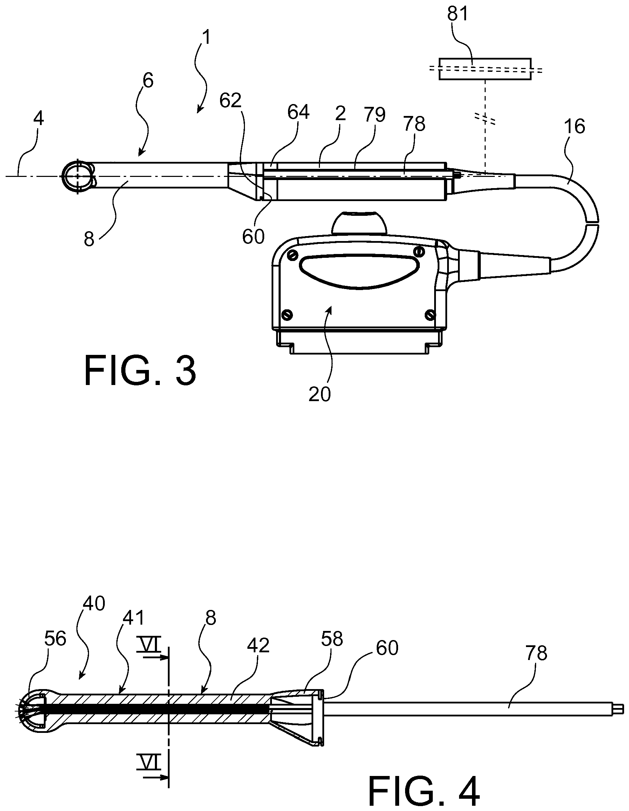

Un câblage électrique 16 est connecté au transducteur 12 et chemine à travers le corps principal 6, puis à travers le manche 2 pour s'extraire à une extrémité arrière de ce dernier. Le câblage électrique 16 est ensuite raccordé à un système d'imagerie à ultrasons 20, représenté sur la figure 3.

Il est noté qu'un ou plusieurs modules d'électronique de multiplexage peuvent être disposés entre les transducteurs 12 et le système d'imagerie 20, afin de réduire le nombre de câbles de liaison du câblage 16. Ces modules peuvent être implantés soit dans le corps principal 10, soit dans le manche 2, sans sortir du cadre de l'invention.

Comme cela a été évoqué ci-dessus, la sonde est équipée de deux dispositifs prenant chacun la forme d'une coque amovible embarquant des moyens optiques. Ces dispositifs 40 vont à présent être détaillés en référence aux figures 4 à 7.

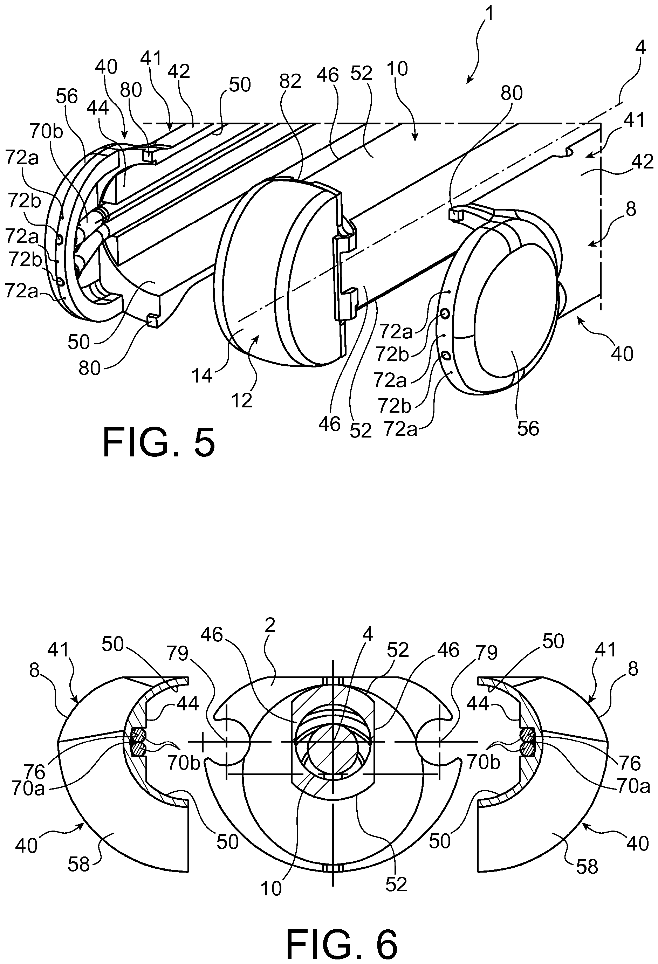

Ils sont agencés autour du corps principal 4, de manière symétrique selon un plan longitudinal passant par l'axe 4. Chacun de ces deux dispositifs 40 présente une coque 41 dont la partie centrale 42 chemine le long du corps principal de sonde 10. Cette partie centrale 42 est cylindrique, d'axe parallèle à l'axe 4. La section droite de cette partie 42 prend la forme globale d'un C dont l'intérieur est orienté vers le corps principal de sonde 10, et dont l'extérieur forme une partie de la surface extérieure lisse 8. Plus précisément, l'intérieur du C présente un méplat 44 destiné à être en contact plan avec une surface plane correspondante 46 du corps principal de sonde 10, comme cela est le mieux visible sur les figures 5 et 6. De plus, les faces intérieures 50 des deux extrémités du C coopèrent avec des surfaces complémentaires cylindriques 52 de section circulaire prévues sur le corps principal de sonde 10, entre les deux surfaces planes 46. Ces coopérations de formes permettent d'orienter correctement les coques 41 par rapport au corps principal de sonde 10.

A l'avant, la coque 41 intègre un élément d'extrémité avant 56 en forme globale de disque, légèrement bombé vers l'extérieur, et destiné à contacter l'un des deux flancs latéraux du transducteur 12. L'axe de ce disque est orthogonal à l'axe 4. Lorsque les deux coques 41 sont assemblées sur la sonde, les deux éléments d'extrémité avant 56 et le transducteur 12 définissent ensemble une extrémité avant de sonde en forme générale de sphère, éventuellement tronquée, ou de cylindre d'axe orthogonal à l'axe 4.

A l'arrière, la coque 41 intègre un élément d'extrémité arrière 58 qui s'évase jusqu'à une surface plane d'extrémité arrière 60, orthogonale à l'axe 4. Cette surface 60 est destinée à être en contact plan avec une surface complémentaire 62 prévue sur une embase élargie 64 du corps principal de sonde 10, montée à l'extrémité avant du manche 2.

Les éléments 42, 56, 58 de la coque 41 sont de préférence réalisés d'une seule pièce, de préférence dans un matériau plastique du type polyuréthane, également biocompatible et assurant l'étanchéité de la sonde.

L'une des particularités de la présente invention réside dans le fait que chaque coque 41 est équipée de moyens optiques embarqués, ici constitués par des fibres optiques cheminant le long de la coque, selon l'axe 4. Il s'agit tout d'abord d'un premier câblage comprenant une pluralité de premières fibres optiques 70a, dont les extrémités avant 72a forment chacune des moyens d'éclairement destinés à assurer un éclairement à l'extérieur de la sonde, en étant montées sur la périphérie avant de l'élément 56.

Il s'agit aussi d'un second câblage comprenant une pluralité de secondes fibres optiques 70b, dont les extrémités avant 72b forment chacune des moyens de collecte destinés à assurer la collecte d'un signal optique produit à l'extérieur de la sonde, par le biais des premières fibres optiques 70a précitées. Ces extrémités avant 72b sont également montées sur la périphérie avant de l'élément 56.

A titre indicatif, les fibres 70a, dite fibres optiques d'excitation, présentent un diamètre gainé de l'ordre de 155 µm, tandis que les fibres 70b, dites fibres de détection, présentent un diamètre gainé de l'ordre de 2,1 mm. Dans le mode de réalisation préféré représenté, chaque coque 41 est équipée de trois premières fibres optiques 70a, ainsi que de deux secondes fibres optiques 70b.

Toutes les extrémités avant 70b, 72b des fibres optiques affleurent la surface extérieure 8 de la coque 41, afin d'éviter des lésions sur les tissus contre lesquels la sonde est destinée à être appliquée. Pour ce faire, ces extrémités avant 70b, 72b traversent l'épaisseur de l'élément 56, pour déboucher de la sonde en direction de l'avant, comme cela est visible sur la figure 5.

Les fibres 70a, 70b s'étendent donc depuis l'extrémité avant de la coque 41, où elles constituent les moyens d'éclairement 72a et les moyens de collecte 72b, puis cheminent vers l'arrière le long de la surface intérieure de la coque, dans une rainure 76 pratiquée sur le méplat 44, comme cela est visible sur la figure 6. Cette rainure 76 permet de guider correctement les fibres optiques 70a, 70b, et de faciliter leur implantation et leur éventuel remplacement.

Ces fibres s'étendent au-delà de l'extrémité arrière de la coque 41, à partir de laquelle elles sont logées dans une gaine 78. Cette gaine est insérée dans une rainure longitudinale 79 pratiquée sur la surface extérieure du manche 2, pour ensuite être raccordées à un système d'imagerie optique 81 représenté schématiquement sur la figure 3, incorporant une source de lumière pulsée ou continue. Cette jonction peut s'effectuer à l'aide de raccords rapides.

Chaque coque 41 présente la particularité de pouvoir être assemblée de manière réversible autour du corps principal de sonde 10, avec ses équipements optiques embarqués. Pour ce faire, elle est équipée de moyens de fixation appropriés, comprenant ici des ergots 80 portés par l'élément d'extrémité avant 56. Plus précisément, l'élément 56 porte deux ergots 80 diamétralement opposés, chacun destiné à être inséré dans une encoche 82 prévue sur l'enveloppe extérieure du transducteur 12. En fait, chacune des deux encoches 82 est prévue pour recevoir deux ergots 80 côte-à-côte, appartenant respectivement aux deux coques 41. Le contact obtenu entre les deux ergots logés dans une encoche, combiné aux contacts de chacun de ces deux ergots avec les flancs latéraux de l'encoche, permettent le maintien des ergots au sein de cette encoche. De plus, le contact plan à l'extrémité arrière des coques, entre les surfaces complémentaires 60, 62, permet de bloquer les coques par rapport au corps principal de sonde.

A cet égard, le montage réversible des coques 41 autour du corps principal de sonde 10 peut donc être assimilé à un clipsage, grâce à l'insertion des ergots dans leurs encoches respectives.

Lorsqu'elles sont assemblées sur la sonde, les coques 41 forment une enveloppe tout autour du corps principal de sonde 10. Cette enveloppe est réalisée par les deux parties centrales 42, ainsi que par les deux éléments d'extrémité arrière 58.

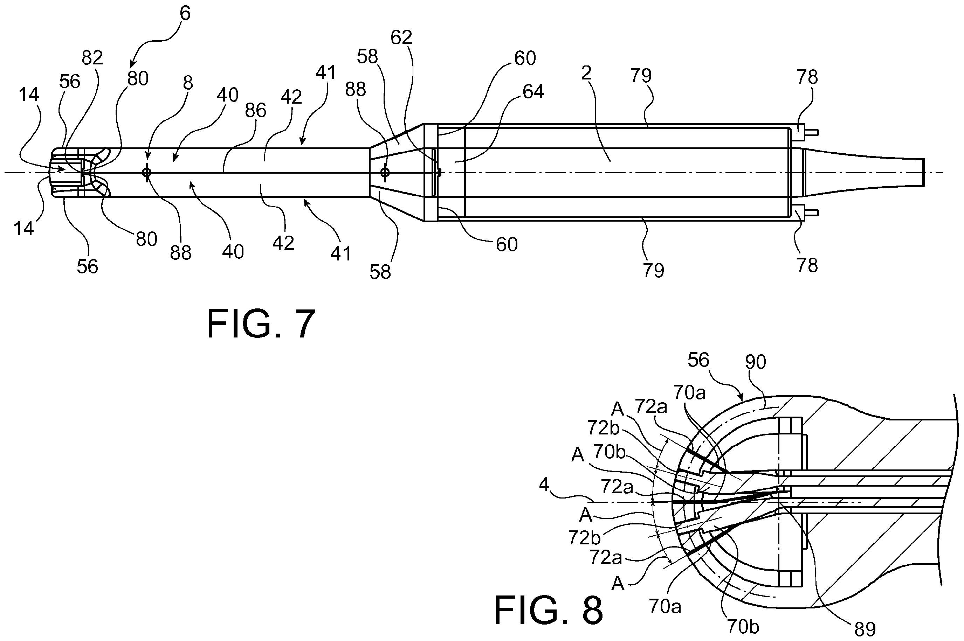

En revanche, ces coques 41 définissent entre les deux éléments d'extrémité avant 56 un espace comblé par le transducteur 12, comme cela est notamment visible sur la figure 7. Sur cette même figure, on peut apercevoir des interfaces de jonction entre les deux coques, référencées 86. Afin d'obtenir une surface extérieure 8 aussi lisse que possible, les rainures créées au niveau de ces interfaces sont comblées par un matériau de remplissage. Cela permet de faciliter la stérilisation de la sonde, sans nécessairement désassembler ses coques à moyens optiques embarqués. Naturellement, ce matériau de remplissage biocompatible, du type silicone / élastomère, est retenu de façon à ne pas trop contraindre un désassemblage ultérieur des coques de la sonde, par exemple réalisé en exerçant un effort séparateur à l'aide de pointes insérées dans des orifices dédiés 88, formés aux interfaces 86 comme cela est visible sur la figure 7. Ce matériau de remplissage est également appliqué aux interfaces entre les coques et le transducteur à ultrasons.

En référence à présent à la figure 8, on peut apercevoir que sur chaque coque, les extrémités avant 72a, 72b des fibres optiques 70a, 70b sont agencées en alternance le long d'un arc de cercle 90 de centre 89, cet arc de cercle 90, de diamètre analogue à celui de l'élément d'extrémité avant 56 traversé par les extrémités avant 72a, 72b des fibres, s'inscrivant dans un plan parallèle à l'axe 4. Lorsque les coques sont assemblées, les deux arcs de cercle 90 se retrouvent agencés parallèlement de part et d'autre du transducteur et de l'axe longitudinal 4.

Par ailleurs, l'écart angulaire A entre l'extrémité avant 72a de chaque première fibre 70a, et chacune des extrémités avant 72b des deux secondes fibres 70b directement consécutives, est de préférence de l'ordre de 15°. Cette valeur d'environ 15° permet d'améliorer considérablement la collecte du signal optique. Les performances de la sonde sont par conséquent sensiblement accrues.

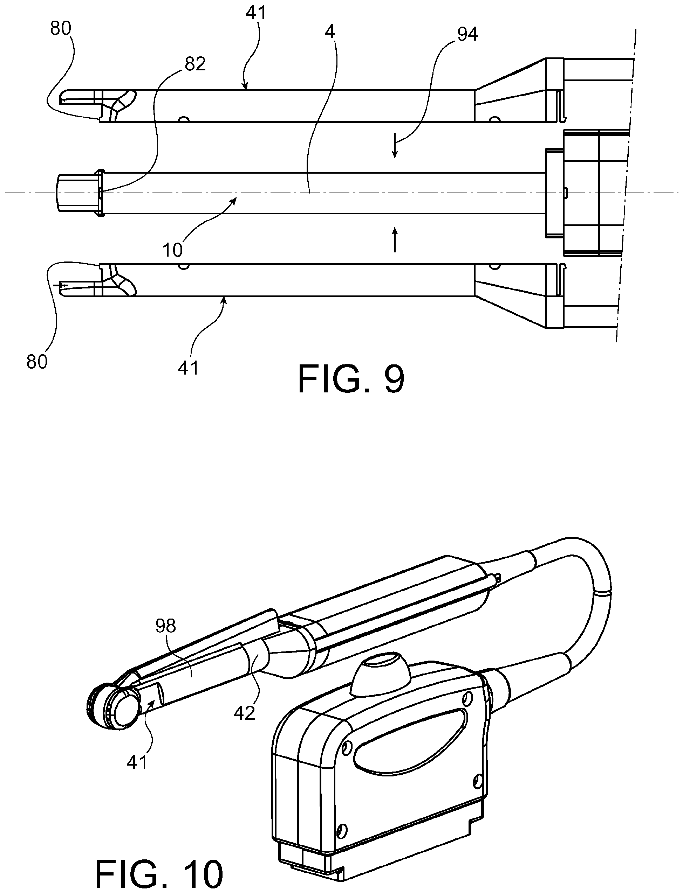

L'invention se rapporte également à un procédé de fabrication de la sonde bimodale qui vient d'être décrite. Ce procédé consiste tout d'abord à équiper chaque coque 41 de ses fibres optiques 70a, 70b, en les insérant latéralement dans leur rainure 76 de la surface intérieure de la coque. Ensuite, ces coques à moyens optiques embarqués sont assemblées, de manière réversible, autour du corps principal de la sonde, comme cela été schématisé sur la figure 9. Ce montage s'effectue en rapprochant les deux coques l'une de l'autre selon une direction 94 orthogonale à l'axe 4, autour du corps principal 10, jusqu'à obtenir le clipsage des ergots 80 dans les encoches 82 correspondantes.

Une fois l'assemblage réversible obtenu, un outil de biopsie 98 destiné à prélever un échantillon de tissu dans la ou les zones suspectes peut être monté extérieurement sur les coques 41, également par clipsage autour des parties centrales 42, comme cela est montré sur la figure 10.

Bien entendu, diverses modifications peuvent être apportées par l'homme du métier à l'invention qui vient d'être décrite, uniquement à titre d'exemple non limitatif.

## Revendications

1. Sonde (1) de diagnostic bimodale à imageries optique et ultrasonore, comportant un corps principal (10) portant à son extrémité avant au moins un transducteur à ultrasons (12), ladite sonde comprenant en outre au moins un dispositif (40) comportant
une coque (41) s'étendant selon une direction longitudinale entre une extrémité arrière et une extrémité avant destinée à former au moins en partie l'extrémité avant de ladite sonde, ledit dispositif comprenant également des moyens d'éclairement (72a) montés sur ladite extrémité avant de la coque de manière à assurer un éclairement à l'extérieur de celle-ci, ainsi que des moyens de collecte ou de détection (72b) montés sur ladite extrémité avant de la coque de manière à assurer la collecte ou la détection d'un signal optique produit extérieurement à ladite coque,
**caractérisée en ce que** ladite coque comporte des moyens de fixation (80, 82) permettant son assemblage réversible autour du corps principal (10) de la sonde, avec lesdits moyens d'éclairement et moyens de collecte ou de détection (72a, 72b) montés sur cette coque.

2. Sonde selon la revendication 1, **caractérisée en ce que** lesdits moyens d'éclairement (72a) sont associés à un premier câblage (70a), **en ce que** lesdits moyens de collecte ou de détection (72b) sont associés à un second câblage (70b), et **en ce que** lesdits premier et second câblages (70a, 70b) cheminent longitudinalement le long de la ladite coque, depuis ladite extrémité avant de la coque, au moins jusqu'à son extrémité arrière.

3. Sonde selon la revendication 2, **caractérisée en ce que** ledit premier câblage comprend au moins une première fibre optique (70a) dont l'extrémité avant (72a), montée sur l'extrémité avant de la coque, forme lesdits moyens d'éclairement.

4. Sonde selon la revendication 2 ou la revendication 3, **caractérisée en ce que** ledit second câblage comprend au moins une seconde fibre optique (70b) dont l'extrémité avant (72b), montée sur l'extrémité avant de la coque, forme lesdits moyens de collecte.

5. Sonde selon l'une quelconque des revendications 2 à 4, **caractérisée en ce que** ledit premier câblage comprend une pluralité de premières fibres optiques (70a) dont chacune des extrémités avant (72a) forme des moyens d'éclairement, **en ce que** ledit second câblage comprend une pluralité de secondes fibres optiques (70b) dont chacune des extrémités avant (72b) forme des moyens de collecte, et **en ce que** les extrémités avant des premières et secondes fibres optiques s'inscrivent sur au moins une ligne courbe (90) .

6. Sonde selon la revendication 5, **caractérisée en ce que** les extrémités avant (72a, 72b) des premières et secondes fibres optiques (70a, 70b) sont agencées en alternance sur une ligne courbe (90).

7. Sonde selon la revendication 5 ou la revendication 6, **caractérisée en ce que** la ligne courbe (90) est un arc de cercle.

8. Sonde selon la revendication 7, **caractérisée en ce que** l'écart angulaire entre l'extrémité avant (72a) d'une première fibre quelconque (70a) et chacune des extrémités avant (72b) des deux secondes fibres (70b) directement consécutives, est compris entre 5° et 25°, de préférence entre 10 et 20°, et est encore plus préférentiellement de l'ordre de 15°.

9. Sonde selon l'une quelconque des revendications précédentes, **caractérisée en ce que** lesdits moyens d'éclairement et lesdits moyens de collecte ou de détection affleurent la surface extérieure (8) de la coque.

10. Sonde selon l'une quelconque des revendications précédentes, **caractérisée en ce qu'**elle comprend au moins deux dispositifs (40), avec les coques (41) formant conjointement une enveloppe tout autour du corps principal de sonde (10), et **en ce qu'**à leurs extrémités avant, lesdites coques (41) définissent un espace comblé par ledit au moins un transducteur à ultrasons (12).

11. Sonde selon la revendication 10, **caractérisée en ce que** le nombre de dispositifs (40) est de deux, les deux coques (41) étant agencées de manière symétrique selon un plan passant par un axe longitudinal (4) de la sonde.

12. Sonde selon la revendication 10 ou la revendication 11, **caractérisée en ce qu'**elle comprend également, aux interfaces (86) entre les coques (41), un matériau de remplissage de ces interfaces.

13. Sonde selon l'une quelconque des revendications 1 à 9, **caractérisée en ce qu'**elle comprend un unique dispositif (40) selon l'une quelconque des revendications 1 à 9, avec une unique coque s'étendant sur 360° autour de l'axe longitudinal (4) de la sonde.

14. Sonde selon l'une quelconque des revendications précédentes, **caractérisée en ce qu'**elle comprend également un manche de préhension (2) à partir duquel le corps principal (10) s'étend vers l'avant, et **en ce que** ledit manche (2) présente une ou plusieurs rainures (79) sur sa surface extérieure, recevant les premier et second câblages (70a, 70b) issus de l'extrémité arrière de la coque (41) de chaque dispositif (40) selon la revendication 2.

15. Sonde selon l'une quelconque des revendications précédentes, **caractérisée en ce qu'**elle est une sonde endorectale ou vaginale.

16. Procédé de fabrication d'une sonde bimodale (1) selon l'une quelconque des revendications précédentes, **caractérisé en ce qu'**il comprend les étapes suivantes :
- équiper la coque (41) de chaque dispositif (40) de ses moyens d'éclairement et moyens de collecte ou de détection (72a, 72b) ; puis
- assembler, de manière réversible, chaque dispositif (40) autour du corps principal de la sonde (10) .

## Patentansprüche

1. Bimodale Diagnosesonde (1) mit Optik- und Ultraschallbildgebung, umfassend einen Hauptkörper (10), der an seinem vorderen Ende wenigstens einen Ultraschallwandler (12) trägt, wobei die Sonde ferner wenigstens eine Vorrichtung (40) umfasst, umfassend:
eine Schale (41), die sich entlang einer Längsrichtung zwischen einem hinteren Ende und einem vorderen Ende erstreckt, und dazu ausgelegt ist, wenigstens teilweise das vordere Ende der Sonde zu bilden, wobei die Vorrichtung ferner Beleuchtungsmittel (72a) umfasst, die an dem vorderen Ende der Schale derart montiert sind, dass eine Beleuchtung außerhalb derselben gewährleistet ist, sowie Auffang- oder Erfassungsmittel (72b), die an dem vorderen Ende der Schale derart montiert sind, dass das Auffangen oder die Erfassung eines optischen Signals gewährleistet ist, das außerhalb der Schale erzeugt ist,
**dadurch gekennzeichnet, dass** die Schale Befestigungsmittel (80, 82) umfasst, die ihre reversible Montage um den Hauptkörper (10) der Sonde herum ermöglichen, wobei die Beleuchtungsmittel und die Auffang- oder Erfassungsmittel (72a, 72b) an dieser Schale montiert sind.

2. Sonde nach Anspruch 1, **dadurch gekennzeichnet, dass** die Beleuchtungsmittel (72a) einer ersten Verkabelung (70a) zugeordnet sind, dass die Auffang- oder Erfassungsmittel (72b) einer zweiten Verkabelung (70b) zugeordnet sind, und dass die erste und zweite Verkabelung (70a, 70b) in Längsrichtung entlang der Schale von dem vorderen Ende der Schale wenigstens bis zu ihrem hinteren Ende verlaufen.

3. Sonde nach Anspruch 2, **dadurch gekennzeichnet, dass** die erste Verkabelung wenigstens eine erste optische Faser (70a) umfasst, deren vorderes Ende (72a), das am vorderen Ende der Schale montiert ist, die Beleuchtungsmittel bildet.

4. Sonde nach Anspruch 2 oder Anspruch 3, **dadurch gekennzeichnet, dass** die zweite Verkabelung wenigstens eine zweite optische Faser (70b) umfasst, deren vorderes Ende (72b), das am vorderen Ende der Schale montiert ist, die Auffangmittel bildet.

5. Sonde nach einem der Ansprüche 2 bis 4, **dadurch gekennzeichnet, dass** die erste Verkabelung eine Mehrzahl von ersten optischen Fasern (70a) umfasst, von denen jedes der vorderen Enden (72a) Beleuchtungsmittel bildet, und dass die zweite Verkabelung eine Mehrzahl von zweiten optischen Fasern (70b) umfasst, von denen jedes der vorderen Enden (72b) Auffangmittel bildet, und dass die vorderen Enden der ersten und zweiten optischen Fasern auf wenigstens einer gekrümmten Linie (90) eingeschrieben sind.

6. Sonde nach Anspruch 5, **dadurch gekennzeichnet, dass** die vorderen Enden (72a, 72b) der ersten und zweiten optischen Fasern (70a, 70b) alternierend auf einer gekrümmten Linie (90) angeordnet sind.

7. Sonde nach Anspruch 5 oder Anspruch 6, **dadurch gekennzeichnet, dass** die gekrümmte Linie (90) ein Kreisbogen ist.

8. Sonde nach Anspruch 7, **dadurch gekennzeichnet, dass** der Winkelabstand zwischen dem vorderen Ende (72a) einer beliebigen ersten Faser (70a) und jedem der vorderen Enden (72b) der zwei direkt anschließenden zweiten Fasern (70b) zwischen 5° und 25° enthalten ist, vorzugsweise zwischen 10 und 20°, und noch weiter bevorzugt in der Größenordnung von 15°.

9. Sonde nach einem der vorhergehenden Ansprüche, **dadurch gekennzeichnet, dass** die Beleuchtungsmittel und die Auffang- oder Erfassungsmittel an der äußeren Oberfläche (8) der Schale anliegen.

10. Sonde nach einem der vorhergehenden Ansprüche, **dadurch gekennzeichnet, dass** sie wenigstens zwei Vorrichtungen (40) umfasst, wobei die Schalen (41) gemeinsam eine Umhüllung um den gesamten Hauptkörper der Sonde (10) herum bilden, und dass die Schalen (41) an ihren vorderen Enden einen Raum definieren, der durch den wenigstens einen Ultraschallwandler (12) gefüllt ist.

11. Sonde nach Anspruch 10, **dadurch gekennzeichnet, dass** die Zahl von Vorrichtungen (40) zwei beträgt, wobei die zwei Schalen (41) symmetrisch zu einer Ebene angeordnet sind, die durch eine Längsachse (4) der Sonde verläuft.

12. Sonde nach Anspruch 10 oder Anspruch 11, **dadurch gekennzeichnet, dass** sie ferner an den Grenzflächen (86) zwischen den Schalen (41) ein Material zum Füllen dieser Grenzflächen umfasst.

13. Sonde nach einem der Ansprüche 1 bis 9, **dadurch gekennzeichnet, dass** sie eine einzige Vorrichtung (40) nach einem der Ansprüche 1 bis 9 umfasst, mit einer einzigen Schale, die sich über 360° um die Längsachse (4) der Sonde erstreckt.

14. Sonde nach einem der vorhergehenden Ansprüche, **dadurch gekennzeichnet, dass** sie ferner einen Griffschaft (2) umfasst, ausgehend von dem sich der Hauptkörper (10) nach vorne erstreckt, und dass der Schaft (2) auf seiner Außenoberfläche eine oder mehrere Rillen (79) aufweist, die die erste und zweite Verkabelung (70a, 70b) aufnehmen, die vom hinteren Ende der Schale (41) jeder Vorrichtung (40) nach Anspruch 2 ausgehen.

15. Sonde nach einem der vorhergehenden Ansprüche, **dadurch gekennzeichnet, dass** sie eine endorektale oder vaginale Sonde ist.

16. Verfahren zur Herstellung einer bimodalen Sonde (1) nach einem der vorhergehenden Ansprüche, **dadurch gekennzeichnet, dass** es die folgenden Schritte umfasst:
- Ausstatten der Schale (41) jeder Vorrichtung (40) mit ihren Beleuchtungsmitteln und Auffang- oder Erfassungsmitteln (72a, 72b); und dann
- Montieren jeder Vorrichtung (40) in reversibler Weise um den Hauptkörper der Sonde (10) herum.

## Claims

1. Bimodal diagnostic probe (1) with optical and ultrasonic imaging comprising a main body (10) with at least one ultrasonic transducer (12) at its front end, said probe also comprising at least one device comprising (40) comprising
a shell (41) extending along a longitudinal direction between a back end and a front end that will at least partly form the front end of said probe, said device also comprising means of illumination (72a) mounted on said front end of the shell so as to provide illumination outside the shell, and collection or detection means (72b) mounted on said front end of the shell so as to collect or detect an optical signal produced outside said shell,
**characterised in that** said shell comprises attachment means (80, 82) so that it can be reversibly assembled around the main body (10) of the probe, with said illumination means and collection or detection means (72a, 72b) mounted on this shell.

2. Probe according to claim 1, **characterised in that** said illumination means (72a) are associated with a first wiring (70a), **in that** said collection or detection means (72b) are associated with a second wiring (70b), and **in that** said first and second wirings (70a, 70b) pass longitudinally along said shell from said front end of the shell, at least as far as its back end.

3. Probe according to claim 2, **characterised in that** said first wiring comprises at least one first optical fibre (70a) the front end (72a) of which, mounted on the front end of the shell, forms said illumination means.

4. Probe according to claim 2 or claim 3, **characterised in that** said second wiring comprises at least one second optical fibre (70b) the front end (72b) of which, mounted on the front end of the shell, forms said collection means.

5. Probe according to any one of claims 2 to 4, **characterised in that** said first wiring comprises a plurality of first optical fibres (70a) the front ends (72a) of each of which form the illumination means, **in that** said second wiring comprises a plurality of second optical fibres (70b) the front ends (72b) of each of which form the collection means, and **in that** the front ends of the first and second optical fibres lie along at least one and preferably only one curved line (90).

6. Probe according to claim 5, **characterised in that** the front ends (72a, 72b) of the first and second optical fibres (70a, 70b) are arranged alternating along a curved line (90).

7. Probe according to claim 5 or claim 6, **characterised in that** the curved line (90) is an arc of a circle.

8. Probe according to claim 7, **characterised in that** the angular difference between the front end (72a) of any first fibre (70a) and each of the front ends (72b) of the two second directly consecutive fibres (70b) is between 5° and 25°, preferably between 10 and 20° and even more preferably of the order of 15°.

9. Probe according to any one of the previous claims, **characterised in that** said illumination means and said collection or detection means are flush with the outer surface (8) of the shell.

10. Probe according to any one of the previous claims, **characterised in that** it comprises at least two devices (40), with the shells (41) forming a casing all around the main body of the probe (10), and **in that** said shells (41) define a space at their front ends filled in by said at least one ultrasonic transducer (12).

11. Probe according to claim 10, **characterised in that** there are two devices (40), the two shells (41) being arranged symmetrically about a plane passing through a longitudinal axis (4) of the probe.

12. Probe according to claim 10 or claim 11, **characterised in that** at the interfaces (86) it comprises a material that fills these interfaces between the shells (41).

13. Probe according to any one of claims 1 to 9, **characterised in that** it comprises a single device (40) according to any one of claims 1 to 9, with one single-piece shell extending around 360° about the longitudinal axis (4) of the probe.

14. Probe according to any one of the previous claims, **characterised in that** it also comprises a gripping handle (2) from which the main body (10) which extends forwards, and **in that** said handle (2) is provided with one or several grooves (79) on its outer surface, into which fit the first and second wirings (70a, 70b) originating from the back end of the shell (41) of each device (40) according to claim 2.

15. Probe according to any one of the previous claims, **characterised in that** it is an endorectal or vaginal probe.

16. Method of fabricating a bimodal probe (1) according to any one of the previous claims, **characterised in that** it includes the following steps:
- equip the shell (41) of each device (40) with its illumination means and collection or detection means(72a, 72b); then
- reversibly assemble each device (40) around the main body of the probe (10).
